(19) 

 **Europäisches Patentamt**
 **European Patent Office**
 **Office européen des brevets**

(11)  **EP 4 181 897 B1**

(12)  **EUROPEAN PATENT SPECIFICATION**

(45) Date of publication and mention
of the grant of the patent:
**17.06.2026 Bulletin 2026/25**

(21) Application number: **21751517.0**

(22) Date of filing: **15.07.2021**

(51) International Patent Classification (IPC):
*A61K 31/05* (2006.01)    *A61K 31/4192* (2006.01)
*A61K 45/06* (2006.01)    *A61P 25/08* (2006.01)

(52) Cooperative Patent Classification (CPC):
(C-Sets available)
**A61K 45/06; A61K 31/4192; A61K 31/658;**
**A61P 25/08**    (Cont.)

(86) International application number:
**PCT/EP2021/069884**

(87) International publication number:
**WO 2022/017949 (27.01.2022 Gazette 2022/04)**

(54) **USE OF CANNABIDIOL IN THE TREATMENT OF SEIZURES ASSOCIATED WITH CHRNA4 MUTATION**

VERWENDUNG VON CANNABIDIOL BEI DER BEHANDLUNG VON MIT CHRNA4-MUTATION
ASSOZIIERTEN ANFÄLLEN

UTILISATION DE CANNABIDIOL DANS LE TRAITEMENT DE CRISES ASSOCIÉES À UNE
MUTATION DE CHRNA4

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **20.07.2020 GB 202011168**

(43) Date of publication of application:
**24.05.2023 Bulletin 2023/21**

(73) Proprietor: **Jazz Pharmaceuticals Research UK**
**Limited**
**Sittingbourne, Kent ME9 8AG (GB)**

(72) Inventors:
• **CHECKETTS, Daniel Adam**
**Sittingbourne, Kent**
**ME9 8AG (GB)**
• **CRAIG, Kevin James**
**Sittingbourne, Kent**
**ME9 8AG (GB)**

(74) Representative: **HGF**
**HGF Limited**
**4th Floor, 1 City Square**
**Leeds LS1 2ES (GB)**

(56) References cited:
**WO-A1-2019/207319    GB-A- 2 531 282**
**GB-A- 2 539 472**

• **BERTRAND D ET AL: "How mutations in the**
**nAChRs can cause ADNFLE epilepsy",**
**EPILEPSIA, RAVEN PRESS LTD, NEW YORK ,**
**US, vol. 43, no. SUPPL. 5, 1 January 2002**
**(2002-01-01), pages 112 - 122, XP002631180,**
**ISSN: 0013-9580**
• **RODRIGUEZ-TIRADO  C S: "Modulation of**
**Nicotinic Receptors by Cannabinoids", 1 April**
**2018 (2018-04-01), Wiley Online Library,**
**XP055851766, Retrieved from the Internet**
**<URL:https://faseb.onlinelibrary.wiley.com/doi/**
**10.1096/fasebj.2018.32.1_supplement.533.78>**
**[retrieved on 20211015]**

**(Cont. next page)**

(52) Cooperative Patent Classification (CPC): (Cont.)

C-Sets
**A61K 31/05, A61K 2300/00;**
**A61K 31/4192, A61K 2300/00**

**Description**

**FIELD OF THE INVENTION**

The present invention relates to the use of cannabidiol (CBD) for the treatment of seizures associated with rare epilepsy syndromes. In particular the seizures associated with rare epilepsy syndromes that are treated are those which are experienced in patients with *CHRNA4* gene mutation. The types of seizures are atonic and myoclonic seizures. Preferably the dose of CBD is between 5 mg/kg/day to 50 mg/kg/day.

In a further embodiment the CBD used is in the form of a highly purified extract of cannabis such that the CBD is present at greater than 95% of the total extract (w/w) and the cannabinoid tetrahydrocannabinol (THC) has been substantially removed, to a level of not more than 0.15% (w/w).

Preferably the CBD used is in the form of a botanically derived purified CBD which comprises greater than or equal to 98% (w/w) CBD and less than or equal to 2% (w/w) of other cannabinoids. More preferably the other cannabinoids present are THC at a concentration of less than or equal to 0.1% (w/w); CBD-C1 at a concentration of less than or equal to 0.15% (w/w); CBDV at a concentration of less than or equal to 0.8% (w/w); and CBD-C4 at a concentration of less than or equal to 0.4% (w/w). The botanically derived purified CBD preferably also comprises a mixture of both trans-THC and cis-THC. Alternatively, a synthetically produced CBD is used.

Most preferably the other cannabinoids present are THC at a concentration of about 0.01% to about 0.1% (w/w); CBD-C1 at a concentration of about 0.1% to about 0.15% (w/w); CBDV at a concentration of about 0.2% to about 0.8% (w/w); and CBD-C4 at a concentration of about 0.3% to about 0.4% (w/w). Most preferably still the THC is present at a concentration of about 0.02% to about 0.05% (w/w).

Where the CBD is given concomitantly with one or more other anti-epileptic drugs (AED), the CBD may be formulated for administration separately, sequentially or simultaneously with one or more AED or the combination may be provided in a single dosage form.

**BACKGROUND TO THE INVENTION**

Epilepsy occurs in approximately 1% of the population worldwide, (Thurman *et al.,* 2011) of which 70% are able to adequately control their symptoms with the available existing anti-epileptic drugs (AED). However, 30% of this patient group, (Eadie *et al.,* 2012), are unable to obtain seizure freedom from the AED that are available and as such are termed as suffering from intractable or "treatment-resistant epilepsy" (TRE).

Intractable or treatment-resistant epilepsy was defined in 2009 by the International League Against Epilepsy (ILAE) as *"failure of adequate trials of two tolerated and appropriately chosen and used AED schedules (whether as monotherapies or in combination) to achieve sustained seizure freedom"* (Kwan *et al.,* 2009).

Individuals who develop epilepsy during the first few years of life are often difficult to treat and as such are often termed treatment resistant. Children who undergo frequent seizures in childhood are often left with neurological damage which can cause cognitive, behavioral and motor delays.

Childhood epilepsy is a relatively common neurological disorder in children and young adults with a prevalence of approximately 700 per 100,000. This is twice the number of epileptic adults per population.

When a child or young adult presents with a seizure, investigations are normally undertaken in order to investigate the cause. Childhood epilepsy can be caused by many different syndromes and genetic mutations and as such diagnosis for these children may take some time.

The main symptom of epilepsy is repeated seizures. In order to determine the type of epilepsy or the epileptic syndrome that a patient is suffering from an investigation into the type of seizures that the patient is experiencing is undertaken. Clinical observations and electroencephalography (EEG) tests are conducted and the type(s) of seizures are classified according to the ILEA classification.

Generalized seizures, where the seizure arises within and rapidly engages bilaterally distributed networks, can be split into six subtypes: tonic-clonic (grand mal) seizures; absence (petit mal) seizures; clonic seizures; tonic seizures; atonic seizures and myoclonic seizures.

Focal (partial) seizures where the seizure originates within networks limited to only one hemisphere, are also split into sub-categories. Here the seizure is characterized according to one or more features of the seizure, including aura, motor, autonomic and awareness / responsiveness. Where a seizure begins as a localized seizure and rapidly evolves to be distributed within bilateral networks this seizure is known as a bilateral convulsive seizure, which is the proposed terminology to replace secondary generalized seizures (generalized seizures that have evolved from focal seizures and are no longer remain localized).

Focal seizures where the subject's awareness / responsiveness is altered are referred to as focal seizures with impairment and focal seizures where the awareness or responsiveness of the subject is not impaired are referred to as focal seizures without impairment.

The *CHRNA4* gene encodes the $\alpha 4$ subunit of a larger protein called a neuronal nicotinic acetylcholine receptor (nAChR), which is made up of a combination of five subunits. nAChR proteins are widely distributed in the brain and act as channels which open in response to chemicals such as acetylcholine and nicotine. Thus, they play an important role in the neuron signaling. nAChR channels affect a wide range of brain functions, including sleep and arousal, fatigue, anxiety, attention, pain perception, and memory. The channels are also active before birth, suggesting they are involved in early brain development.

Mutations in the *CHRNA4* gene have been identified in people with autosomal dominant nocturnal frontal lobe epilepsy (ADNFLE).

D. Bertrand, et al., Epilepsia, 2002, 43, 112-122, analysed the functional properties of four nAChR mutants associated with ADNFLE. Their findings indicated that a gain of function in these mutant receptors may be at the origin of the neuronal network dysfunction that causes the epileptic seizures.

Autosomal dominant nocturnal frontal lobe epilepsy (ADNFLE) is an uncommon, inherited form of epilepsy characterized by seizures occurring at night during sleep. Some people with ADNFLE also have seizures during the day. These seizures can last from a few seconds to a few minutes, and can vary from causing simple arousal from sleep, to dramatic muscle spasms and movements.

Most people with ADNFLE have normal intellect, but some affected people have reduced intellect. Some people with ADNFLE also have psychiatric disorders or behavior problems, but it is unclear if these are directly related to ADNFLE. The condition is inherited in an autosomal dominant manner and may be caused by a mutation in any of several genes.

The onset of ADNFLE ranges from infancy to adulthood, but most cases begin in childhood and episodes tend to become milder and less frequent with age.

Treatment of ADNFLE involves anti-seizure medications to control seizures. Carbamazepine is usually used as the main drug but oxcarbamazepine, topiramate and acetazolamide may also be used as add-on therapies.

Cannabidiol (CBD), a non-psychoactive derivative from the cannabis plant, has demonstrated anti-convulsant properties in several anecdotal reports, pre-clinical and clinical studies both in animal models and humans. Three randomized control trials showed efficacy of the purified pharmaceutical formulation of CBD in patients with Dravet and Lennox-Gastaut syndrome.

Based on these three trials, a botanically derived purified CBD preparation was approved by FDA in June 2018 for the treatment of seizures associated with Dravet and Lennox-Gastaut syndromes.

Documents including WO2020/109806, GB2531282 and GB2531278 disclose the effectiveness of highly purified CBD in the treatment of seizures associated with epileptic syndromes such as Lennox-Gastaut Syndrome. However, there is no data of patients with CHRNA4-related epilepsy nor is there any mention of this condition.

C. S. Rodriguez-Tirado, et al., The FASEB Journal, 2018, 32, 533.78 (Experimental Biology 2018 Meeting Abstract) describes a project aimed at characterizing the effects of CBD and THC on the function of the $\alpha 7$ nAChR. The results indicates that CBD reduces the response of the $\alpha 7$ nAChR to ACh by 49% (p value=0.0003).

The applicant has found by way of an open label, expanded-access program that treatment with CBD resulted in a significant reduction in atonic and myoclonic seizures in patients with *CHRNA4* mutation.

## BRIEF SUMMARY OF THE DISCLOSURE

In accordance with a first aspect of the present invention there is provided a cannabidiol (CBD) preparation for use in the treatment of seizures in patients with a *CHRNA4* mutation, wherein the seizures are atonic or myoclonic seizures.

In a further embodiment, the CBD preparation comprises greater than 95% (w/w) CBD and not more than 0.15% (w/w) tetrahydrocannabinol (THC).

Preferably the CBD preparation comprises greater than or equal to 98% (w/w) CBD and less than or equal to 2% (w/w) other cannabinoids, wherein the less than or equal to 2% (w/w) other cannabinoids comprise the cannabinoids tetrahydrocannabinol (THC); cannabidiol-C1 (CBD-C1); cannabidivarin (CBDV); and cannabidiol-C4 (CBD-C4), and wherein the THC is present as a mixture of trans-THC and cis-THC.

Preferably the CBD preparation is used in combination with one or more concomitant anti-epileptic drugs (AED).

Preferably the one or more AED is rufinamide.

In one embodiment the CBD is present is isolated from cannabis plant material. Preferably at least a portion of at least one of the cannabinoids present in the CBD preparation is isolated from cannabis plant material.

In a further embodiment the CBD is present as a synthetic preparation. Preferably at least a portion of at least one of the cannabinoids present in the CBD preparation is prepared synthetically.

Preferably the dose of CBD is greater than 5 mg/kg/day. More preferably the dose of CBD is 20 mg/kg/day. More preferably the dose of CBD is 25 mg/kg/day. More preferably the dose of CBD is 50 mg/kg/day.

## DEFINITIONS

Definitions of some of the terms used to describe the invention are detailed below:

Over 100 different cannabinoids have been identified, see for example, Handbook of Cannabis, Roger Pertwee, Chapter 1, pages 3 to 15. These cannabinoids can be split into different groups as follows: Phytocannabinoids; Endocannabinoids and Synthetic cannabinoids (which may be novel cannabinoids or synthetically produced phytocannabinoids or endocannabinoids).

"Phytocannabinoids" are cannabinoids that originate from nature and can be found in the cannabis plant. The phytocannabinoids can be isolated from plants to produce a highly purified extract or can be reproduced synthetically.

"Highly purified cannabinoids" are defined as cannabinoids that have been extracted from the cannabis plant and purified to the extent that other cannabinoids and non-cannabinoid components that are co-extracted with the cannabinoids have been removed, such that the highly purified cannabinoid is greater than or equal to 95% (w/w) pure.

"Synthetic cannabinoids" are compounds that have a cannabinoid or cannabinoid-like structure and are manufactured using chemical means rather than by the plant.

Phytocannabinoids can be obtained as either the neutral (decarboxylated form) or the carboxylic acid form depending on the method used to extract the cannabinoids. For example, it is known that heating the carboxylic acid form will cause most of the carboxylic acid form to decarboxylate into the neutral form.

"Treatment-resistant epilepsy" (TRE) or "intractable epilepsy" is defined as per the ILAE guidance of 2009 as epilepsy that is not adequately controlled by trials of one or more AED.

"Tonic-clonic seizures" consist of two phases: the tonic phase and the clonic phase. In the tonic phase the body becomes entire rigid, and in the clonic phase there is uncontrolled jerking. Tonic-clonic seizures may or may not be preceded by an aura, and are often followed by headache, confusion, and sleep. They may last mere seconds or continue for several minutes. These seizures are also known as a grand mal seizure.

"Atonic seizures" occur when a person suddenly loses muscle tone so their head or body may go limp. They are also known as drop attacks. In some children, only their head drops suddenly. They can begin in one area or side of the brain (focal onset) or both sides of the brain (generalized onset).

"Myoclonic seizures" are characterised by a 'muscle jerk'. Myoclonic seizures are brief but can happen in clusters (many happening close together in time) and often happen shortly after waking. In myoclonic seizures the person is conscious, but they are classified as generalised seizures.

"Absence seizures" also may be called "petit mal" seizures. These types of seizure cause a loss of awareness for a short time. They mainly affect children although can happen at any age. During an absence seizure, a person may: stare blankly into space; look like they're "daydreaming"; flutter their eyes; make slight jerking movements of their body or limbs. The seizures usually only last up to 15 seconds and may occur several times a day.

## DETAILED DESCRIPTION

### PREPARATION OF HIGHLY PURIFIED CBD EXTRACT

The following describes the production of the highly-purified (>95% w/w) cannabidiol extract which has a known and constant composition.

In summary the drug substance used is a liquid carbon dioxide extract of high-CBD containing chemotypes of *Cannabis sativa* L. which had been further purified by a solvent crystallization method to yield CBD. The crystallisation process specifically removes other cannabinoids and plant components to yield greater than 95% CBD. Although the CBD is highly purified because it is produced from a cannabis plant rather than synthetically there is a small number of other cannabinoids which are co-produced and co-extracted with the CBD. Details of these cannabinoids and the quantities in which they are present in the medication are as described in Table A below.

**Table A: Composition of highly purified CBD extract**

| Cannabinoid | Concentration |
|---|---|
| CBD | > 95% w/w |
| CBDA | NMT 0.15% w/w |
| CBDV | NMT 1.0% w/w |
| $\Delta^9$ THC | NMT 0.15% w/w |

(continued)

| Cannabinoid | Concentration |
|---|---|
| CBD-C4 | NMT 0.5% w/w |
| > - greater than<br>NMT - not more than | |

## PREPARATION OF BOTANICALLY DERIVED PURIFIED CBD

The following describes the production of the botanically derived purified CBD which comprises greater than or equal to 98% w/w CBD and less than or equal to other cannabinoids was used in the open label, expanded-access program described in Example 1 below.

In summary the drug substance used in the trials is a liquid carbon dioxide extract of high-CBD containing chemotypes of *Cannabis sativa* L. which had been further purified by a solvent crystallization method to yield CBD. The crystallisation process specifically removes other cannabinoids and plant components to yield greater than 95% CBD w/w, typically greater than 98% w/w.

The *Cannabis sativa* L. plants are grown, harvested, and processed to produce a botanical extract (intermediate) and then purified by crystallization to yield the CBD (botanically derived purified CBD).

The plant starting material is referred to as Botanical Raw Material (BRM); the botanical extract is the intermediate; and the active pharmaceutical ingredient (API) is CBD, the drug substance.

All parts of the process are controlled by specifications. The botanical raw material specification is described in Table B and the CBD API is described in Table C.

**Table B: CBD botanical raw material specification**

| Test | Method | Specification |
|---|---|---|
| Identification:<br>-A<br>-B<br>-C | <br>Visual<br>TLC<br>HPLC/UV | <br>Complies<br>Corresponds to standard (for CBD & CBDA)<br>Positive for CBDA |
| Assay:<br>CBDA + CBD | In-house (HPLC/UV) | NLT 90% of assayed<br>cannabinoids by peak area |
| Loss on Drying | Ph. Eur. | NMT 15% |
| Aflatoxin | UKAS method | NMT 4ppb |
| Microbial:<br>- TVC<br>- Fungi<br>- E.coli | Ph.Eur. | NMT $10^7$cfu/g<br>NMT $10^5$cfu/g<br><br>NMT $10^2$cfu/g |
| Foreign Matter: | Ph.Eur. | NMT 2% |
| Residual Herbicides and Pesticides | Ph.Eur. | Complies |

**Table C: Specification of an exemplary botanically derived purified CBD preparation**

| Test | Test Method | Limits |
|---|---|---|
| Appearance | Visual | Off-white / pale yellow crystals |
| Identification A | HPLC-UV | Retention time of major peak corresponds to certified CBD Reference Standard |
| Identification B | GC-FID/MS | Retention time and mass spectrum of major peak corresponds to certified CBD Reference Standard |
| Identification C | FT-IR | Conforms to reference spectrum for certified CBD Reference Standard |
| Identification D | Melting Point | 65 - 67°C |

(continued)

| Test | Test Method | Limits |
|---|---|---|
| Identification E | Specific Optical Rotation | Conforms with certified CBD Reference Standard; -110° to -140° (in 95% ethanol) |
| Total Purity | Calculation | ≥ 98.0% |
| Chromatographic Purity 1 | HPLC-UV | ≥ 98.0% |
| Chromatographic Purity 2 | GC-FID/MS | ≥ 98.0 % |
| CBDA CBDV THC CBD-C4 | HPLC-UV | NMT 0.15% w/w 0.2-1.0% w/w 0.01-0.1% w/w 0.3-0.5% w/w |
| Residual Solvents: Alkane Ethanol | GC | NMT 0.5% w/w NMT 0.5% w/w |
| Residual Water | Karl Fischer | NMT 1.0% w/w |

The purity of the botanically derived purified CBD preparation was greater than or equal to 98%. The botanically derived purified CBD includes THC and other cannabinoids, e.g., CBDA, CBDV, CBD-C1, and CBD-C4.

In some embodiments, the CBD preparation comprises not more than 0.15% THC based on total amount of cannabinoid in the preparation. In some embodiments, the CBD preparation comprises about 0.01% to about 0.1% THC based on total amount of cannabinoid in the preparation. In some embodiments, the CBD preparation comprises about 0.02% to about 0.05% THC based on total amount of cannabinoid in the preparation.

In some embodiments, the CBD preparation comprises about 0.2% to about 1.0% CBDV based on total amount of cannabinoid in the preparation. In some embodiments, the CBD preparation comprises about 0.2% to about 0.8% CBDV based on total amount of cannabinoid in the preparation.

In some embodiments, the CBD preparation comprises about 0.3% to about 0.5% CBD-C4 based on total amount of cannabinoid in the preparation. In some embodiments, the CBD preparation comprises about 0.3% to about 0.4% CBD-C4 based on total amount of cannabinoid in the preparation.

In some embodiments, the CBD preparation comprises about 0.1% to about 0.15% CBD-C1 based on total amount of cannabinoid in the preparation.

Distinct chemotypes of the *Cannabis sativa* L. plant have been produced to maximize the output of the specific chemical constituents, the cannabinoids. Certain chemovars produce predominantly CBD. Only the (-)-trans isomer of CBD is believed to occur naturally. During purification, the stereochemistry of CBD is not affected.

*Production of CBD botanical drug substance*

An overview of the steps to produce a botanical extract, the intermediate, are as follows:

a) Growing
b) Direct drying
c) Decarboxylation
d) Extraction - using liquid $CO_2$
e) Winterization using ethanol
f) Filtration
g) Evaporation

High CBD chemovars were grown, harvested, dried, baled and stored in a dry room until required. The botanical raw material (BRM) was finely chopped using an Apex mill fitted with a 1 mm screen. The milled BRM was stored in a freezer prior to extraction.

Decarboxylation of CBDA to CBD was carried out using heat. BRM was decarboxylated at 115°C for 60 minutes.

Extraction was performed using liquid $CO_2$ to produce botanical drug substance (BDS), which was then crystalized to produce the test material. The crude CBD BDS was winterized to refine the extract under standard conditions (2 volumes of ethanol at -20°C for approximately 50 hours). The precipitated waxes were removed by filtration and the solvent was removed to yield the BDS.

*Production of botanically derived purified CBD preparation*

The manufacturing steps to produce the botanically derived purified CBD preparation from BDS were as follows:

a) Crystallization using $C_5$-$C_{12}$ straight chain or branched alkane
b) Filtration
c) Vacuum drying

The BDS produced using the methodology above was dispersed in $C_5$-$C_{12}$ straight chain or branched alkane. The mixture was manually agitated to break up any lumps and the sealed container then placed in a freezer for approximately 48 hours. The crystals were isolated via vacuum filtration, washed with aliquots of cold $C_5$-$C_{12}$ straight chain or branched alkane, and dried under a vacuum of <10mb at a temperature of 60°C until dry. The botanically derived purified CBD preparation was stored in a freezer at -20°C in a pharmaceutical grade stainless steel container, with FDA food grade approved silicone seal and clamps.

*Physicochemical properties of the botanically derived purified CBD*

The botanically derived purified CBD used in the clinical trial described in the invention comprises greater than or equal to 98% (w/w) CBD and less than or equal to 2% (w/w) of other cannabinoids. The other cannabinoids present are THC at a concentration of less than or equal to 0.1% (w/w); CBD-C1 at a concentration of less than or equal to 0.15% (w/w); CBDV at a concentration of less than or equal to 0.8% (w/w); and CBD-C4 at a concentration of less than or equal to 0.4% (w/w).

The botanically derived purified CBD used additionally comprises a mixture of both trans-THC and cis-THC. It was found that the ratio of the trans-THC to cis-THC is altered and can be controlled by the processing and purification process, ranging from 3.3:1 (trans-THC:cis-THC) in its unrefined decarboxylated state to 0.8:1 (trans-THC:cis-THC) when highly purified.

Furthermore, the cis-THC found in botanically derived purified CBD is present as a mixture of both the (+)-cis-THC and the (-)-cis-THC isoforms.

Clearly a CBD preparation could be produced synthetically by producing a composition with duplicate components.

Example 1 below describes the use of a botanically derived purified CBD in an open label, expanded-access program to investigate the clinical efficacy and safety of purified pharmaceutical cannabidiol formulation (CBD) in the treatment of seizures associated with CHRNA4 mutation.

**EXAMPLE 1: CLINICAL EFFICACY AND SAFETY OF PURIFIED PHARMACEUTICAL CANNABIDIOL (CBD) IN THE TREATMENT OF PATIENTS DIAGNOSED WITH *CHRNA4* MUTATION**

*Study design*

The subject was required to be on one or more AEDs at stable doses for a minimum of two weeks prior to baseline and to have stable vagus nerve stimulation (VNS) settings and ketogenic diet ratios for a minimum of four weeks prior to baseline.

The patient was administered botanically derived purified CBD in a 100 mg/mL sesame oil-based solution. Dose was increased to a goal of 25 mg/kg/day.

A maximum dose of 50 mg/kg/day could be utilised for the patient if they were tolerating the medication but had not achieved seizure control; the patient had further weekly titration by 5mg/kg/day.

There was one patient in this study, and they received CBD for 24 weeks. Modifications were made to concomitant AEDs as per clinical indication.

Seizure frequency, intensity, and duration were recorded by caregivers in a diary during a baseline period of at least 28 days. Changes in seizure frequency relative to baseline were calculated after at least 2 weeks and at defined timepoints of treatment.

*Statistical Methods:*

Patients may be defined as responders if they had more than 50% reduction in seizure frequency compared to baseline. The percent change in seizure frequency was calculated as follows:

$$\text{\% change seizure frequency} = \frac{((\text{weekly seizure frequency } \textit{time interval}) - (\text{weekly seizure frequency } \textit{Baseline})) \times 100}{(\text{weekly seizure frequency } \textit{Baseline})}$$

The percent change of seizure frequency may be calculated for any time interval where seizure number has been recorded. For the purpose of this example the percent change of seizure frequency for the end of the treatment period was calculated as follows:

$$\text{\% reduction seizure frequency} = \frac{((\text{weekly seizure frequency } \textit{Baseline}) - (\text{weekly seizure frequency } \textit{End})) \times 100}{(\text{weekly seizure frequency } \textit{Baseline})}$$

**Results**

*Patient description*

One patient enrolled in the open label, expanded-access program had *CHRNA4* mutation. The patient experienced several different seizure types including tonic-clonic, atonic, myoclonic and absence seizures and was taking one concomitant AED.
The patient was 7 years old and he was male as detailed in Table 1 below.

**Table 1: Patient demographics, seizure type and concomitant medication**

| Patient Number | Age (years) | Sex | Seizure types | Concomitant AEDs |
|---|---|---|---|---|
| 1 | 7.45 | M | Tonic-clonic, atonic, myoclonic, absence | RFN |
| RFN = rufinamide | | | | |

*Study medication and concomitant medications*

The patient on the study was titrated up to 25 mg/kg/day of CBD.
The patient was on one concomitant AED at the time of starting CBD.

*Clinical changes*

Table 2 illustrates the seizure frequency for the patient as well as the dose of CBD given.

**Table 2: Seizure frequency data for Patient 1**

| Patient 1 | | | | | |
|---|---|---|---|---|---|
| Time | Seizure Type | | | | Dose CBD (mg/kg/day) |
| | Tonic-clonic | Atonic | Myoclonic | Absence | |
| Baseline | 0.4 | 2800.0 | 2800.0 | 0.0 | - |
| 4 weeks | 1.0 | 2800.0 | 2800.0 | 0.0 | 15.0 |
| 8 weeks | 0.0 | 2800.0 | 2800.0 | 0.0 | 25.0 |
| 12 weeks | 1.0 | 2561.0 | 2560.0 | 0.0 | 25.0 |
| 16 weeks | 3.0 | 1260.0 | 840.0 | 84.0 | 25.0 |
| 24 weeks | 18.7 | 840.0 | 840.0 | 140.0 | 23.8 |

Patient 1 was treated for 24 weeks and experienced a 70% reduction in atonic and myoclonic seizures over the treatment period.
Overall, CBD was effective in reducing the frequency of atonic and myoclonic seizures.

**Conclusions**

These data indicate that CBD was able to significantly reduce the number of seizures associated with *CHRNA4* mutation. Clearly the treatment is of significant benefit in this difficult to treat epilepsy syndrome given the high response rate experienced in the patient.

In conclusion, this study signifies the use of CBD for treatment of seizures associated with *CHRNA4* mutation. Seizure types include atonic and myoclonic seizures for which seizure frequency rates decreased by significant rates, by 70%.

**Claims**

1. A cannabidiol (CBD) preparation for use in the treatment of seizures in patients with a *CHRNA4* mutation, wherein the seizures are atonic or myoclonic seizures.

2. A CBD preparation for use according to claim 1, wherein the CBD preparation comprises greater than 95% (w/w) CBD and not more than 0.15% (w/w) tetrahydrocannabinol (THC).

3. A CBD preparation for use according to any preceding claim, wherein the CBD preparation comprises greater than or equal to 98% (w/w) CBD and less than or equal to 2% (w/w) other cannabinoids, wherein the less than or equal to 2% (w/w) other cannabinoids comprise the cannabinoids tetrahydrocannabinol (THC); cannabidiol-C1 (CBD-C1); cannabidivarin (CBDV); and cannabidiol-C4 (CBD-C4), and wherein the THC is present as a mixture of trans-THC and cis-THC.

4. A CBD preparation for use according to any preceding claim, wherein the CBD preparation is used in combination with one or more concomitant anti-epileptic drugs (AED).

5. A CBD preparation for use according to claim 4, wherein the one or more AED is rufinamide.

6. A CBD preparation for use according to any preceding claim, wherein the CBD is isolated from cannabis plant material.

7. A CBD preparation for use according to any preceding claim, wherein at least a portion of at least one of the cannabinoids present in the CBD preparation is isolated from cannabis plant material.

8. A CBD preparation for use according to any one of claims 1 to 5, wherein the CBD is present as a synthetic preparation.

9. A CBD preparation for use according to claim 8, wherein at least a portion of at least one of the cannabinoids present in the CBD preparation is prepared synthetically.

10. A CBD preparation for use according to any preceding claim, wherein the dose of CBD is greater than 5 mg/kg/day.

11. A CBD preparation for use according to any preceding claim, wherein the dose of CBD is 20 mg/kg/day.

12. A CBD preparation for use according to any one of claims 1 to 10, wherein the dose of CBD is 25 mg/kg/day.

13. A CBD preparation for use according to any one of claims 1 to 10, wherein the dose of CBD is 50 mg/kg/day.

**Patentansprüche**

1. Cannabidiol- (CBD-)Zubereitung zur Verwendung bei der Behandlung von Anfällen bei Patienten mit einer CHRNA4-Mutation, wobei die Anfälle atonische oder myoklonische Anfälle sind.

2. CBD-Zubereitung zur Verwendung gemäß Anspruch 1, wobei die CBD-Zubereitung mehr als 95 % (Gew.-%) CBD und nicht mehr als 0,15 % (Gew.-%) Tetrahydrocannabinol (THC) umfasst.

3. CBD-Zubereitung zur Verwendung gemäß einem der vorhergehenden Ansprüche, wobei die CBD-Zubereitung mehr als oder gleich 98 % (Gew.-%) CBD und weniger als oder gleich 2 % (Gew.-%) andere Cannabinoide umfasst, wobei die weniger als oder gleich 2 % (Gew.-%) anderen Cannabinoide die Cannabinoide Tetrahydrocannabinol (THC); Cannabidiol-C1 (CBD-C1); Cannabidivarin (CBDV); und Cannabidiol-C4 (CBD-C4) umfassen, und wobei das THC als ein Gemisch von trans-THC und cis-THC vorliegt ist.

4. CBD-Zubereitung zur Verwendung gemäß einem der vorhergehenden Ansprüche, wobei die CBD-Zubereitung in Kombination mit einem oder mehreren begleitenden antiepileptischen Arzneimitteln (AED) verwendet wird.

5. CBD-Zubereitung zur Verwendung gemäß Anspruch 4, wobei das eine oder die mehreren AED Rufinamid ist.

6. CBD-Zubereitung zur Verwendung gemäß einem der vorhergehenden Ansprüche, wobei das CBD aus Cannabispflanzenmaterial isoliert ist.

7. CBD-Zubereitung zur Verwendung gemäß einem der vorhergehenden Ansprüche, wobei zumindest ein Teil von zumindest einem der in der CBD-Zubereitung vorliegenden Cannabinoide aus Cannabispflanzenmaterial isoliert ist.

8. CBD-Zubereitung zur Verwendung gemäß einem der Ansprüche 1 bis 5, wobei das CBD als eine synthetische Zubereitung vorliegt.

9. CBD-Zubereitung zur Verwendung gemäß Anspruch 8, wobei zumindest ein Teil von zumindest einem der Cannabinoide, die in dem CBD-Zubereitung vorhanden sind, synthetisch zubereitet ist.

10. CBD-Zubereitung zur Verwendung gemäß einem der vorhergehenden Ansprüche, wobei die Dosis von CBD größer als 5 mg/kg/Tag ist.

11. CBD-Zubereitung zur Verwendung gemäß einem der vorhergehenden Ansprüche, wobei die Dosis von CBD 20 mg/kg/Tag ist.

12. CBD-Zubereitung zur Verwendung gemäß einem der Ansprüche 1 bis 10, wobei die Dosis von CBD 25 mg/kg/Tag ist.

13. CBD-Zubereitung zur Verwendung gemäß einem der Ansprüche 1 bis 10, wobei die Dosis von CBD 50 mg/kg/Tag ist.

**Revendications**

1. Préparation de cannabidiol (CBD) destinée à être utilisée dans le traitement de crises chez des patients présentant une mutation de *CHRNA4,* dans laquelle les crises sont des crises atoniques ou myocloniques.

2. Préparation de CBD destinée à être utilisée selon la revendication 1, dans laquelle la préparation de CBD comprend plus de 95 % (p/p) de CBD et pas plus de 0,15 % (p/p) de tétrahydrocannabinol (THC).

3. Préparation de CBD destinée à être utilisée selon une quelconque revendication précédente, dans laquelle la préparation de CBD comprend au moins 98 % (p/p) de CBD et au plus 2 % (p/p) d'autres cannabinoïdes, dans laquelle les autres cannabinoïdes présents à au plus 2 % (p/p) comprennent les cannabinoïdes tétrahydrocannabinol (THC); cannabidiol-C1 (CBD-C1) ; cannabidivarine (CBDV) ; et cannabidiol-C4 (CBD-C4), et dans laquelle le THC est présent sous forme d'un mélange de trans-THC et de cis-THC.

4. Préparation de CBD destinée à être utilisée selon une quelconque revendication précédente, dans laquelle la préparation de CBD est utilisée en combinaison avec un ou plusieurs médicaments antiépileptiques (AED) concomitants.

5. Préparation de CBD destinée à être utilisée selon la revendication 4, dans laquelle le ou les AED sont le rufinamide.

6. Préparation de CBD destinée à être utilisée selon une quelconque revendication précédente, dans laquelle le CBD est isolé à partir de matière végétale de cannabis.

7. Préparation de CBD destinée à être utilisée selon une quelconque revendication précédente, dans laquelle au moins une partie d'au moins l'un des cannabinoïdes présents dans la préparation de CBD est isolée à partir de matière végétale de cannabis.

8. Préparation de CBD destinée à être utilisée selon l'une quelconque des revendications 1 à 5, dans laquelle le CBD est présent sous forme d'une préparation synthétique.

9. Préparation de CBD destinée à être utilisée selon la revendication 8, dans laquelle au moins une partie d'au moins l'un des cannabinoïdes présents dans la préparation de CBD est préparée de manière synthétique.

10. Préparation de CBD destinée à être utilisée selon une quelconque revendication précédente, dans laquelle la dose de CBD est supérieure à 5 mg/kg/jour.

11. Préparation de CBD destinée à être utilisée selon une quelconque revendication précédente, dans laquelle la dose de CBD est de 20 mg/kg/jour.

12. Préparation de CBD destinée à être utilisée selon l'une quelconque des revendications 1 à 10, dans laquelle la dose de CBD est de 25 mg/kg/jour.

13. Préparation de CBD destinée à être utilisée selon l'une quelconque des revendications 1 à 10, dans laquelle la dose de CBD est de 50 mg/kg/jour.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2020109806 A
- GB 2531282 A
- GB 2531278 A

### Non-patent literature cited in the description

- **D. BERTRAND et al.** *Epilepsia*, 2002, vol. 43, 112-122
- **C. S. RODRIGUEZ-TIRADO et al.** *The FASEB Journal*, 2018, vol. 32 (533), 78
- **ROGER PERTWEE**. Handbook of Cannabis